Europäisches Patentamt

European Patent Office

Office européen des brevets

(19)

(11) Publication number: 0 333 474
A2

(12) **EUROPEAN PATENT APPLICATION**

(21) Application number: 89302603.9

(22) Date of filing: 16.03.89

(51) Int. Cl.⁴: **C 07 K 3/18**
C 12 P 21/02

(30) Priority: 17.03.88 JP 62007/88

(43) Date of publication of application:
20.09.89 Bulletin 89/38

(84) Designated Contracting States:
CH DE FR GB IT LI SE

(71) Applicant: MITSUI TOATSU CHEMICALS, Inc.
2-5 Kasumigaseki 3-chome
Chiyoda-Ku Tokyo 100 (JP)

(72) Inventor: Morii, Mitsuyoshi
Mitsui Toatsu Apt. 2-4-5 1541, Yabecho Totsuka-ku
Yokohama-shi Kanagawa-ken (JP)

Ohoka, Masaharu
Mitsui Toatsu Apt. 2-22 2882, Iijimacho Sakae-ku
Yokohama-shi Kanagawa-ken (JP)

Mori, Kunizo
Mitsui Toatsu Apt. 4-43 2882, Iijimacho Sakae-ku
Yokohama-shi Kanagawa-ken (JP)

Kawashima, Nobuhiro
8587-3, Tana
Sagamihara-shi Kanagawa-ken (JP)

(74) Representative: Stuart, Ian Alexander et al
MEWBURN ELLIS & CO. 2/3 Cursitor Street
London EC4A 1BQ (GB)

(54) **Process for the selective removal of endotoxin.**

(57) Endotoxin contaminants are selectively removed from an aqueous solution of a valuable protein by contacting the solution with an adsorbent capable of specifically adsorbing the protein thereon, the adsorbent is washed with a solution containing an amino compound to selectively remove only the endotoxin, and the protein is eluted from the adsorbent.

EP 0 333 474 A2

## Description

### Process for the Selective Removal of Endotoxin

This invention relates to a process for the separation of an endotoxin, and specifically to a process for the selective removal of an endotoxin to produce a preparation of a physiologically-active high-molecular weight substance, which is suitable for injection or parenteral administration.

The present invention is useful in the course of purification of a valuable protein such as a physiologically-active high-molecular weight substance. This invention can be practised preferably by using any one of various chromatographic techniques such as hydrophobic chromatography, affinity chromatography, adsorption chromatography, and metal chelate chromatography.

So-called pyrogens are known as substances which cause the body temperature of a homoiotherm to rise abnormally even when present at extremely small, trace levels.

Known pyrogens include bacterial substances, inflammatory substances, plant polysaccharides, blood group specific substances, etc. Of these, bacterial substances are most troublesome and are called "bacterial toxins". They are generally divided into exotoxins and endotoxins. Among these bacterial toxins, the endotoxin composed principally of lipopolysaccharides derived from cell walls of a gram-negative bacterium, that is, a so-called ohne Hauch antigen (O antigen) has the strongest pyrogenicity and moreover, once it is mixed with the physiologically-active protein, its removal is very difficult.

Current therapeutics include physiologically-active protein substances produced by recombinant DNA technology or cell fusion technology. Usable host cells include Escherichia coli, Bacillus subtilis, yeast and animal cells. Mixing of one or more pyrogens with the desired cells is often observed in the course of collection of or isolation and purification of these starting materials.

It is a reality of endotoxin contamination of parenteral pharmaceutical products that one or more endotoxins are detected from about 50% of distilled water and physiological saline products for injection, and in the case of transfusion preparations, at least one endotoxin is detected from as many as 90% of them.

The formulation of physiologically-active high-molecular weight substances into pharmaceutical preparations requires a high-degree of concentration in many instances in the course of the formulation. Even when the concentration of an endotoxin in a starting solution is at an extremely low level, it is similarly concentrated in the final concentrated preparation. Injection of the endotoxin therefore results in the undesired side effect of fever. It is thus very important to either remove or inactivate these endotoxins.

Prior methods for the removal or inactivation of endotoxins, include inter alia inactivation with heat, an acid, an alkali or the like; to treat an endotoxin-containing injection or the like with a surfactant such as "Triton X-100" (trade name, product of Rohm & Haas Co.); to physically remove endotoxins by ultrafiltration; to subject endotoxins to oxidative treatment with a permanganate salt or hydrogen peroxide; and to remove endotoxins by absorption on activated carbon or an ion-exchange resin.

Prior procedures often inactivate or remove the physiologically active substance and/or introduce an undesired contaminant into the parenteral product. The method involving the use of heat, an acid, an alkali or the like has the serious drawback that a physiologically-active substance, effective as a principal active component, may also be decomposed, denatured or inactivated in the process. Use of a surfactant, for example as in U.S. Patent No. 4,412,985, has a low endotoxin-removing effect for physiologically-active substances which react with endotoxins to bind them. Moreover, the surfactant itself is a foreign material in the case of parenterals, such as injections and the like, so that the removal of the surfactant is indispensable. This method is therefore impractical. The molecular weights of endotoxins are about 8,000 daltons. The molecular weights of their polymers, however, reach as high as about 1,000,000 daltons. Most of physiologically- active substances have a molecular weight in this range. Ultrafiltration is thus incapable of completely removing endotoxins whose molecular weights range widely, as mentioned above. Oxidative treatment also shares a potential problem that the physiologically-active substance may also be inactivated in the process. Further, the oxidizing agent itself is a foreign material which should not be introduced into the pharmaceutical product. This method is hence not preferred. Adsorption on a resin or the like has the drawback that the active protein itself may also be adsorbed. It has hence been difficult to achieve selective removal of an endotoxin by such conventional methods.

It is desirable to achieve one or more of the following:
to provide a process for selectively removing an endotoxin from an aqueous solution of a valuable protein substance contaminated with an endotoxin, particularly parenteral aqueous solutions containing a high-molecular weight protein as the active agent;
to provide a process for removing an endotoxin without impairing the activity of the associated valuable protein substance;
to provide a process which is adapted to remove endotoxins from an endotoxin-contaminated aqueous solution of a valuable protein substance that is free of the drawbacks described above.

With the foregoing in view, the present inventors have carried out various investigations. As a result, it has been found that an amino compound, for example, arginine, benzamidine, urea, guanidine hydrochloride, and the like, interacts with endotoxins. The present inventors have also found a process for removing an endotoxin using the above phenomenon, leading to completion of this invention.

The inventors found that it is beneficial to bring an aqueous solution containing valuable protein substance and contaminated with an endotoxin into contact with an adsorbent capable of specifically adsorbing the protein substance thereon thereby to have the protein adsorbed on the adsorbent, washing the adsorbent with a solution containing an amino compound thereby to selectively remove the endotoxin, and then eluting the substance freed of the endotoxin, from the adsorbent.

The term "valuable protein substance" as used herein means a physiologically active product, notably a protein, that is formulasted for parenteral administration. As illustrative examples of the valuable protein substance, may therefore be mentioned various hormones such as insulin and growth hormone, enzymes such as urokinase and tissue plasminogen activators, proteolytic enzyme inhibitors such as α-1-proteinase inhibitor, hirudin and cystatin, serum proteins such as albumin and γ-globulin, antiviral proteins such as interferon, etc.

No particular limitation is imposed on the concentration of the valuable protein substance. Any concentration may be used so long as the valuable protein substance can be dissolved.

As an adsorbent useful in the practice of this invention, may be mentioned by way of example an ordinary chromatographic carrier bearing a substance which reacts specifically with and binds the protein to the carrier. In addition, hydroxyapatite is also usable.

By the term "substance which reacts specifically with the protein", hereinafter called a "ligand", includes antibody proteins directed against the valuable protein substances; lectins such as concanavalin A; enzyme inhibitors employed upon purification of their corresponding enzymes; enzymes used upon purification of their corresponding proteolytic enzyme inhibitors; reactive dyes, such as Cibacron Blue (trade name; product of Ciba-Geigy Co.) and Procion Red (trade name; product of ICI Co.); compounds containing one or more hydrophobic groups, such as phenyl and/or octyl groups; and compounds containing one or more groups capable of forming a complex with metal ions, especially divalent metal ions such as zinc ions or cupric ions, etc.

Suitable carriers useful for carrying the ligand include conventional chromatographic carriers, such as insoluble agarose, dextran, cellulose, polyacrylamide, polyethylene glycol glycidyl-methacrylate polymer, divinylbenzene-crosslinked polystyrene and glass beads.

Specific ligand-carrying carriers, which may be mentioned by way of example include phenyl-Sepharose, octyl-Sepharose, zinc-chelate-Sepharose, blue-Sepharose, red-Sepharose, concanavalin-A-agarose, and the like. Their commercial products can be used.

It is also possible to use a ligand after having a carrier attached to it by a method known per se in the art. The method of joining the ligand to carrier may be any suitable known method depending on the type of ligand and carrier. These methods are described in detail, for example, in "Method in ENZYMOLOGY" 34, 13-108, eds, W.B. Jakoby and M. Wilcheck, Academic Press, N.Y. (1974).

No particular limitation is imposed on the manner for bringing the valuable protein substance into contact with the adsorbent to have the former adsorbed on the latter. A routine method may be employed, for example, using an operation in which contact and adsorption is effected by a batch method or column method. In the case of the column method, the solution may be fed as either an ascending flow or a descending flow. The operational temperature should fall within a range in which the valuable protein substance remains stable, and preferably, within a range of from a temperature at which the aqueous medium does not freeze to 25°C.

The washing solution useful in the practice of this invention is a solution of a suitable amino compound in an aqueous medium such as water. Illustrative examples of the amino compound include arginine; benzamidine; urea; guanidine hydrochloride; basic amino acids such as lysine, ornithine and ε-aminocapronic acid; amines such as ethylamine, ethylenediamine, ethanolamine and hydroxylamine; amides such as acetamide and benzamide; amidines such as p-aminobenzamidine; urea derivatives such as methylurea and ethylurea; etc. Examples are argine, benzamidine, urea and guanidine hydrochloride. Using an amino compound as a washing solution permits not only the removal of the free endotoxin but also the selective desorption and removal of the endotoxin exclusively, even when the latter endotoxin is bound partly to the valuable protein substance adsorbed on the carrier.

The concentration of the amino compound may vary depending on the type of the amino compound used, the type of the ligand employed, the type of chromatography applied, etc. The concentration may generally be in a range of 1-500 mM for arginine and in a range of 1-200 mM for benzamidine as two examples. However, where there is a potential problem; in the case of urea, guanidine hydrochloride or the like, the amino compound may irreversibly denature the protein as the valuable protein substance and/or ligand, so it is used at a concentration low enough to avoid such modification. For example, urea may generally be used in a range of from 10 mM to 8 M, preferably 0.1-4 M, and more preferably 0.5-2 M, while guanidine hydrochloride may be used normally in a range of from 10 mM to 6 M, and preferably from 50 mM to 2 M.

Conditions under which the adsorbent is washed with the solution containing the amino compound vary depending on the type of chromatography used and the type of the protein to be obtained. Conditions of operation should be determined while paying attention to avoid elution of the desired valuable protein substance using empeical or trial-and-error procedures until the appropriate conditions are ascertained.

For example, in affinity chromatography in which an antibody for a valuable protein substance is used as a ligand, the pH may be in a range of 4-11, preferably 5-10, and an amino compound is used at concentration in the above-described range. On the other hand, in affinity chromatography in which a proteolytic enzyme is used as a ligand for its corresponding inhibitor or a proteolytic enzyme inhibitor is employed as a ligand for its corresponding proteolytic enzyme, the pH may be in a range of 5-10, preferably 6-9, and the the amino compound is used at a concentration in a range of 1-500 mM in the case of arginine, in a range of 1-50 mM in the case of benzamidine, in a range of from 10 mM to 2 M, more preferably from 0.1 to 1 M in the case of urea, or in a range of from 10 mM to 2 M, preferably from 50 mM to 1 M in the case of guanidine hydrochloride.

In affinity chromatography using a lectin or an analogous substance, a pH in the neighborhood of the neutral level, for example, in a range of from about 6 to about 9, may be used in general. The amino compound may be used at a concentration of 1-500 mM for arginine, 1-200 mM for benzamidine or 10 mM to 2 M for urea and guanidine hydrochloride, by way of example.

In adsorption chromatography, for example, when hydroxyapatite is used, a pH around the neutral level, for example, in a range of from about 6 to about 8 may be used. The amino compound may be used at a concentration of 1-100 mM for arginine and benzamidine or 10-500 mM for urea and guanidine hydrochloride, by way of example.

In hydrophobic chromatography, an acidic-to-neutral pH, for example, a pH of from about 2 to about 8 may be used. The amino compound may be used at a concentration of 1-100 mM for arginine and benzamidine or from 10 mM to 2 M for urea and guanidine hydrochloride, by way of example.

In metal chelate chromatography, a pH around the neutral level, for example, a pH of from about 6 to about 9 may be used. The amino compound may be used at a concentration of 1-500 mM for arginine, 1-200 mM for benzamidine or from 10 mM to 2 M for urea and guanidine hydrochloride by way of example.

The volume of the washing solution used may be 1-50 times, preferably, 10-20 times the volume of the adsorbent in each type of chromatography.

By the above operation, the endotoxin is selectively removed and, moreover, other foreign proteins adsorbed on the adsorbent.

The valuable protein substance desired is then eluted with an eluent. A suitable eluent may be chosen depending on the substance desired. For example, in affinity chromatography using an antibody, a solution having a low pH of from about 2 to 4 or a solution of a salt containing chaotropic ions such as a thiocyanate salt can be used. In affinity chromatography using a proteolytic enzyme or a proteolytic enzyme inhibitor, a solution having a low pH of from about 2 to about 5, a solution of a salt containing chaotropic ions such as a thiocyanate salt, or a solution containing a reagent capable of competitively acting on an enzyme-inhibiting reaction can be used. In affinity chromatography relying upon a lectin or the like, elution can be achieved by using a solution of a saccharide such as α-methyl-D-glucoside as is employed routinely.

In adsorption chromatography, for example, when hydroxyapatite is used, elution can be conducted at a pH around the neutral level, for example, a pH of from about 6 to about 8 and at an increased salt or phosphate ion concentration or with a solution whose pH ranges from 8 to 11.

In hydrophobic chromatography, elution can be conducted with a solution having a pH of at least the neutral level, a solution containing a salt at a low concentration or a solution containing an organic solvent.

In metal chelate chromatography, elution can be effected with a solution having a low pH, for example, in a range of from about 2 to about 5 or with a solution of a reagent having high chelation-forming ability.

The present invention will hereinafter be described by the following non-limiting Examples. The detection of each endotoxin was conducted in accordance with the Limulus test (Haemostasis, $\underline{7}$, 183, 1987).

Example 1:

After culturing Bowes melanoma cells (ATCC CRL 1424 G361) in a tissue culture medium "RPMI-1640" nourished with 10% of fetal bovine serum inactivated thermally at 56° C for 30 minutes, the resultant culture was washed once, followed by further culture in a serum-free medium for 24 hours. The medium was collected to obtain a culture fluid containing tissue plasminogen activator (t-PA). Ten liters of the culture fluid was partly purified by a suitable method to provide an aqueous t-PA solution as a starting material. The pH and activity of the solution were 7-8 and $1 \times 10^4$ IU/m$\ell$, respectively. The endotoxin content of the solution was found to be about 20 ng/$\ell$ (determined using E. coli 055:B5 as a standard reference) by the Limulus test.

Twenty milliliters of the solution were caused to flow through a column packed with 2 m$\ell$ of ETI-agarose (5 mg ETI/m$\ell$ resin) which had been prepared by immobilizing on an agarose resin Erythrina trypsin inhibitor (ETI) purified in accordance with the method proposed by F.J. Joubert et al in Hoppe-Selers Z. Physiol Chem, $\underline{362}$, 531-538 (1981).

Subsequently, the column was washed with 20 m$\ell$ of a 50 mM NaH$_2$PO$_4$-NaOH aqueous solution (pH 9.5) containing 0.5 M of arginine and 0.1 M of sodium chloride, and then with 10 m$\ell$ of distilled water. The t-PA activity in the washings was about 5% of the total activity applied.

t-PA was then eluted with 5 m$\ell$ of a 0.1 M NaH$_2$PO$_4$-H$_3$PO$_4$ aqueous solution (pH 2.8) containing 0.1 M of sodium chloride, whereby adsorbed t-PA was successfully recovered almost quantitatively. The endotoxin content was not higher than 10 pg/m$\ell$ as measured by the Limulus test, so that a substantially endotoxin-free preparation was obtained.

Example 2:

A solution prepared in a similar manner as in Example 1 was used as a starting material. Twenty milliliters of the solution was caused to flow through a column packed with 50 m$\ell$ of α-tPA agarose (10 mg α-tPA/m$\ell$ resin) which had been prepared by immobilizing anti-human t-PA monoclonal antibody on agarose. The column was thereafter washed with 200 mℓ of a 0.05 M phosphate buffer (pH 7.5) containing 0.1 M of benzamidine and 1 M of sodium chloride and then with 100 mℓ of distilled water.

Substantially no t-PA activity was detected from the washings. Then, t-PA was eluted with 50 mℓ of a 0.1 M $NaH_2PO_4$-$H_3PO_4$ aqueous solution (pH 2.5) containing 0.1 M of sodium chloride. About 85% of the total amount of the t-PA applied was recovered. Further, no endotoxin was observed in the eluate by the Limulus test.

Example 3:
An aqueous t-PA solution prepared in a similar manner as in Example 1 was used as a starting material. Twenty milliliters of the solution was caused to flow through a column packed with 50 mℓ of zinc-chelate-Sepharose which had been equilibrated with a 20 mM Tris-HCl buffer (pH 7.5) containing 1.0 M of sodium chloride. The column was then washed with 500 mℓ of a 20 mM Tris-HCl buffer (pH 7.5) containing 0.1 M of arginine and 1 M of sodium chloride.

The t-PA activity in the washing was about 5% of the total activity applied. After washing the column with 100 mℓ of distilled water, t-PA was eluted with 200 mℓ of a 0.1 M $NaH_2PO_4$-$H_3PO_4$ aqueous solution (pH 2.5) containing 0.1 M of sodium chloride. About 80% of the total amount of the t-PA applied was recovered. Further, no endotoxin was observed in the eluate by the Limulus test.

Comparative Example 1:
An experiment was conducted in a similar manner as in Example 3, except that 500 mℓ of a 20 mM Tris-HCl buffer (pH 7.5) containing 1 M sodium chloride, which was free of arginine, was used as a washing solution.

Although t-PA was recovered at substantially the same recovery rate as in Example 3, the endotoxin content of the eluate was found to be about 60 pg/mℓ (determined using E. coli 055:B5 as a standard reference) by the Limulus test.

Example 4:
In 5 mℓ of a 0.02 M phosphate buffer (pH 6.0) containing 0.1 M of sodium chloride, was dissolved 10 mg of human serum albumin (product of Sigma Chemical Company). The endotoxin content of the solution was found to be about 20 ng/mℓ (determined using E. coli as a standard reference) by the Limulus test.

The above solution was caused to flow through a column packed with 2 mℓ of "Red-Sepharose" (trade name; product of Pharmacia Fine Chemicals Co.) which had been equilibrated with the above buffer. Thereafter, the column was washed with 20 mℓ of the above buffer containing 0.5 M of urea and then with 20 mℓ of the above buffer. The column was then eluted with 10 mℓ of a 0.05 M $NaH_2PO_4$-NaOH aqueous solution (pH 9.5) containing 1.0 M sodium chloride. The recovery rate of the protein was about 90%. The endotoxin content was found to be not higher than 10 pg/mℓ by the Limulus test.

Example 5:
A solution of inter-α-trypsin inhibitor solution, which had been obtained by subjecting human plasma to partial purification by a suitable method, in a 0.05M phosphate buffer (pH 6.8) containing 3 M of sodium chloride was used as a starting material. The inter-α-trypsin inhibitor content of the solution was found to be 0.7 mg/mℓ as measured by an enzyme immunoassay (EIA). On the other hand, the endotoxin content was found to be about 40 ng/mℓ (determined using E. coli 055: B5 as a standard reference) by the Limulus test.

Three milliliters of the solution was caused to flow through a column packed with 5 mℓ of phenyl-Sepharose, followed by washing of the column with 50 mℓ of a 0.05 M phosphate buffer (pH 6.8) containing 0.5 M of guanidine hydrochloride and 3 M of sodium chloride and then with 50 mℓ of a 0.05 M phosphate buffer (pH 6.8) containing 3 M of sodium chloride only. The inter-α-trypsin inhibitor content of the washings was about 10% of the total amount of the inhibitor applied.

Inter-α-trypsin inhibitor was then eluted with 20 mℓ of a 0.05 M $Na_2HPO_4$ aqueous solution (pH 9.5). About 80% of the total amount of the inter-α-trypsin inhibitor applied was recovered. The endotoxin content of the eluate was found to be not higher than 10 pg/mℓ by the Limulus test.

**Claims**

1. A process for selectively removing an endotoxin from an endotoxin-contaminated solution of a desired valuable protein, which comprises (a) contacting an endotoxin-contaminated aqueous solution of a desired protein with an adsorbent capable of specifically adsorbing the protein thereon thereby absorbing the protein on the adsorbent, (b) washing the adsorbent with a solution containing an amino compound thereby to selectively remove the endotoxin while the protein remains adsorbed on the adsorbent, and then (c) eluting the substance from the adsorbent.

2. The process as claimed in Claim 1, wherein the amino compound is arginine, benzamidine, urea or guanidine hydrochloride.

3. The process as claimed in Claim 1, wherein the solution containing an amino compound is an arginine solution of 1-500 mM concentration.

4. The process as claimed in Claim 3, wherein the concentration is of 1-100 mM concentration.

5. The process as claimed in Claim 1, wherein the solution containing an amino compound is a benzamidine solution of 1-200 mM concentration.

6. The process as claimed in Claim 5, wherein the concentration is 1-50 mM.

7. The process as claimed in Claim 1, wherein the solution containing an amino compound is an urea solution of 10 mM - 8 M concentration.

8. The process as claimed in Claim 7, wherein the concentration is 10-500 mM.

9. The process as claimed in Claim 1, wherein the solution containing an amino compound is a guanidine hydrochloride solution of 10 mM - 6 M concentration.

10. The process as claimed in claim 9, wherein the concentration is 10-500 mM.